# EUROPEAN PATENT APPLICATION

(11) **EP 1 291 411 A1**
(43) Date of publication of application: **12.03.2003**
(21) Application number: 01307618.7
(22) Date of filing: 07.09.2001
(51) Int. Cl.: C12M 1/00

(54) **Incubator**

(71) Applicant: The Automation Partnership (Cambridge) Limited, Royston, Hertfordshire SG8 5WY (GB)
(72) Inventor: Bargh, Adrian, London N8 7LS (GB); Grant, Paul Simon, Cambridge CB1 2DY (GB); Crooks, David Alan, Cambridge CB1 6YN (GB)
(74) Representative: Haley, Stephen

(57) **Abstract**

An incubator comprising a housing and base defining a sealed incubation region. There is also means for heating the incubation region in use. A sample receiving member defines a slot for receiving, in use, a sample therein, the sample receiving member is arranged such that, in use, the slot can be moved from a position external to the incubator to a position in the incubation region without passing through a position in which there is a direct opening between the incubating region and the exterior of the incubator. A sample receiving mechanism is positioned within the incubating region for receiving from the sample receiving slot, when it is in incubating region, a sample in use and moving that sample to a storage region within the incubation region.

## Description

This invention relates to an incubator and, particularly, to an incubator for use in the incubation and growth of live cells in an automated system.

Incubators are well known. For many years incubators have been a necessary part in the culture of live cells for use in biochemical and chemical analysis. Many live cells can only be grown at a temperature and humidity levels that are either above or below ambient temperatures. Furthermore, many cells require their surrounding temperature and humidity levels to be kept within very specific ranges to ensure fast and efficient growth.

Many known incubators are capable of providing the appropriate temperature and humidity ranges necessary for cell growth. However, few have the ability to be accessed by automated machinery. Furthermore, most have associated with them problems associated with access in that they have no means for limiting contamination from their external environment when they are accessed for cell cultures either to be placed in them or removed from them. Such contamination can include the introduction of large volumes of atmospheric air or other gasses at a temperature other than that which is optimum for cell growth, as well as the possibility of contaminating microbes and particulates entering them. The contaminating microbes are a particular problem, as they can result in fungal growth which can result in contamination of the cell cultures as well as affecting the overall efficiency of the incubator.

The present invention seeks to overcome some of the above problems.

According to the present invention there is provided an incubator comprising:
a housing and base defining a sealed incubation region;
means for heating the incubation region in use;
a sample receiving member defining a slot for receiving, in use, a sample therein, the sample receiving member being arranged such that, in use, the slot can be moved from a position external to the incubator to a position in the incubation region without passing through a position in which there is a direct opening between the incubation region and the exterior of the incubator; and
a sample receiving mechanism positioned within the incubation region for receiving from the sample receiving slot, when it is in incubation region, a sample in use and moving that sample to a storage region within the incubation region.

The sample receiving member may be rotatable so that the slot can rotate from the external position to the incubation region position. In this case, there may be two or more slots positioned around the receiving member. The housing may have an access port for allowing manual access to the incubator region for maintenance.

The sample storage region may comprise a series of rotatable sample holders, each of which may be removable. The sample handling device may comprise an automated clamping head that is movable along at least two axes.

The incubator may also comprise means for controlling the humidity to the incubation region.

With the present invention it is possible for an incubator to be provided for which no human access is required during standard operation and which is hermetically sealed so that internal temperatures and humidities can be maintained to a high degree of accuracy, it also enables a high degree of control of the gas composition in the incubator as well as ensuring the risk of contamination from external contaminants is reduced to a minimum.

An example of the present invention will now be described with reference to the accompanying drawings, in which:

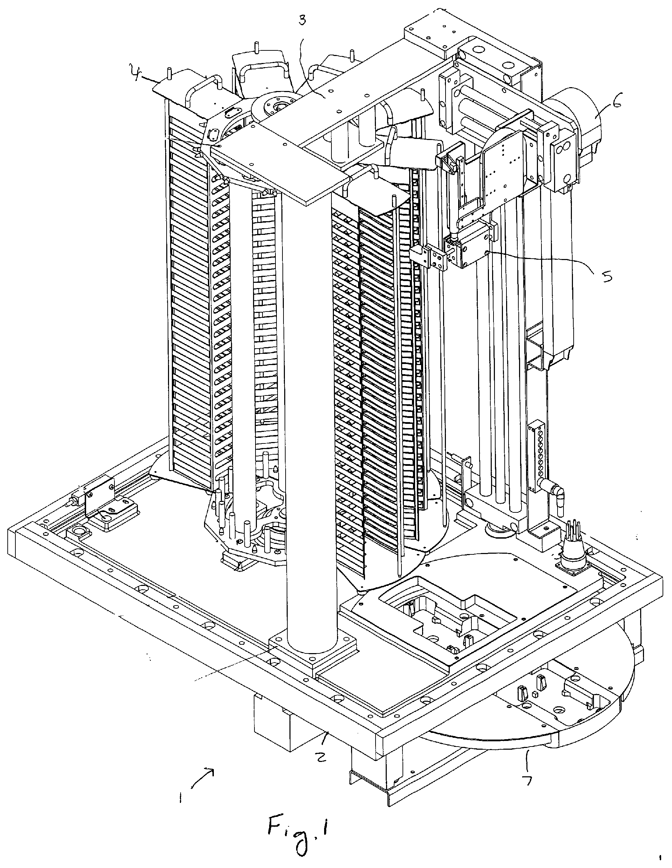

Figure 1 is a perspective view of the internal construction of an incubator according to the present invention.

Referring to figure 1, an incubator 1 has a base 2 which, in use, is connected to a housing (not shown). The housing may be formed as a solid casing that can be removed from the base 2 for maintenance or may have a maintenance door as part of it to enable a manual operator to gain access for maintenance. The housing also has associated therewith the necessary heating and humidity control apparatus, together with associated insulation, to ensure that the environment within the housing and base 2 is kept at the values appropriate for the particular type of incubation being performed. The humidity control may be a heated source of liquid (not shown) in the base 2.

Supported on the base 2 is a carousel 3 of optionally independently removable incubator tray holders 4. The carousel 3 can be rotated by a motor (not shown) so that automated rotation of the carousel 3 can control the positions of the tray holders 4 within the incubator 1.

Also supported on the base 2 is an automated tray clamp 5 which is driven by a motor 6 so that the tray clamp 5 can be moved in a direction parallel to the rotational axis of the carousel 3 to access individual trays within a selected tray holder 4. The clamp 5 can also move in a direction perpendicular to the rotational axis of the carousel 3 so that it can engage with a selected tray within a selected tray holder 4 and remove the selected tray.

Positioned within the base 2 is a rotatable tray part 7 which is positioned along the axis of movement of the tray clamp 5 so that a tray obtained by the tray clamp 5 can be placed therein in use.

In operation, when a tray is required to be removed from the incubator 1 its associated tray holder 4 can be rotated on the carousel 3 until it is aligned with the axis of the tray clamp 5. The tray clamp 5 can then be moved into alignment with the selected tray and then into engagement with it to remove the tray from the holder 4. The tray clamp 5 then places the tray into the rotating tray receiving port 7 in the base 2. The rotating tray receiving slot 7 is then rotated so that the tray is moved out of the incubator 1 to a position where it can either be accessed by a human operator or by additional automated machinery. The tray receiving port 7 preferably has two receiving openings for trays so that when one is rotated out from the incubator 1 another is rotated into the incubator 1. In this way, if it is necessary also to insert a tray into the incubator 1 during a tray removal process then the process can be speeded up. As the tray receiving port 7 is rotating the tray clamp 5 in a port is positioned to pickup any inserted tray from the port 7 and place it in a selected position in a selected tray holder 4 in the carousel 3.

The present invention allows total automation of the tray accessing and insertion process for the incubator 1. By provision of a simple rotating port arrangement 7 (or a reciprocating slot arrangement as an alternative) the interior of the incubator one is not exposed to large volumes of external gasses that would affect the internal temperature and humidity and gas environment of the incubator 1. There is also a considerable reduction in the likely risk of contamination that constant access by a standard opening door would provide.

## Claims

1. An incubator comprising:
a housing and base defining a sealed incubation region;
heating means for heating the incubation region in use;
a sample receiving member defining a slot for receiving, in use, a sample therein, the sample receiving member being arranged such that, in use, the slot can be moved from a position external to the incubator to a position in the incubation region without passing through a position in which there is a direct opening between the incubation region and the exterior of the incubator; and
a sample receiving mechanism positioned within the incubation region for receiving from the sample receiving slot, when it is in incubation region, a sample in use and moving that sample to a storage region within the incubation region.

2. An incubator according to claim 1, wherein the sample receiving member is rotatable so that the slot can rotate from the external position to the incubation region position.

3. An incubator according to claim 2, wherein there are two or more slots positioned around the receiving member.

4. An incubator according to any preceding claim, wherein the housing has an access port for allowing manual access to the incubation region for maintenance.

5. An incubator according any preceding claim, wherein the sample storage region comprises a series of rotatable sample holders.

6. An incubator according to claim 5, wherein the sample holders are removable.

7. An incubator according to any preceding claim, wherein the sample receiving mechanism comprises an automated clamping head that is movable along at least two axes.

8. An incubator according to any preceding claim, further comprising means for controlling the humidity in the incubation region.
